# EUROPÄISCHE PATENTSCHRIFT

(11) **EP 2 027 102 B1**
(45) Veröffentlichungstag und Bekanntmachung des Hinweises auf die Patenterteilung: **21.04.2010**
(21) Anmeldenummer: 07729283.7
(22) Anmeldetag: 18.05.2007
(51) Int. Cl.: C07D 307/89

(54) **HERSTELLUNG VON PHTHALSÄUREANHYDRID DURCH GASPHASENOXIDATION VON O-XYLOL IN EINEM HAUPT- UND NACHREAKTOR**
PRODUCTION OF PHTHALIC ANHYDRIDE BY GAS PHASE OXIDATION OF o-XYLOL IN A PRIMARY AND A SECONDARY REACTOR
PRODUCTION D'ANHYDRIDE PHTALIQUE PAR OXYDATION EN PHASE GAZEUSE D'O-XYLOL DANS UN RÉACTEUR PRINCIPAL ET UN RÉACTEUR SECONDAIRE

(30) Priorität: 19.05.2006 EP 06010416
(43) Veröffentlichungstag der Anmeldung: 25.02.2009
(73) Patentinhaber: BASF SE, 67056 Ludwigshafen (DE)
(72) Erfinder: MACKEWITZ, Thomas, 68219 Mannheim (DE); ROSOWSKI, Frank, 68239 Mannheim (DE); TENTEN, Andreas, 67434 Neustadt/weinstrasse (DE)
(86) Internationale Anmeldenummer: PCT/EP2007/054839
(87) Internationale Veröffentlichungsnummer: WO 2007/135102

(56) Entgegenhaltungen:
- WO-A-02/16299
- WO-A-03/070680
- DE-A1- 2 005 969
- DE-A1- 10 144 857
- DE-A1- 19 807 018

## Beschreibung

Die vorliegende Erfindung betrifft ein Verfahren zur Herstellung von Phthalsäureanhydrid durch Gasphasenoxidation von o-Xylol in einem Hauptreaktor und einem nachgeschalteten Nachreaktor.

Die Gasphasenoxidation von o-Xylol und/oder Naphthalin zu Phthalsäureanhydrid (PSA) ist wohl bekannt und in der Literatur vielfach beschrieben. Eine Übersicht gibt beispielsweise H. Suter, Phthalsäureanhydrid und seine Verwendung, Steinkopf Verlag, Darmstadt, 1972 oder F.K. Towae, W. Enke, R. Jäckh, N. Bhargava in Ullmann's Encyclopedia of Industrial Chemistry, Vo. A20, 1992, S. 181- 190.

Im Allgemeinen erfolgt die Oxidationsreaktion von o-Xylol an Luft in einem mit einer Salzschmelze temperierten Rohrbündelreaktor. Dabei wird ein praktisch vollständiger Umsatz des eingesetzten Kohlenwasserstoffs im Reaktor und gleichzeitig eine hohe Produktqualität des gebildeten Phthalsäureanhydrids angestrebt. Um dies einerseits zu erreichen und gleichzeitig jedoch eine Schädigung des Katalysators durch hohe Hotspot-Temperaturen zu vermeiden, werden im Allgemeinen unterschiedlich aktive Katalysatoren schichtweise in den Rohren angeordnet, wobei in der Regel der weniger aktive Katalysator so im Festbett angeordnet wird, dass das Reaktionsgemisch mit ihm als erstes in Kontakt kommt, während sich der aktivste Katalysator zum Gasaustritt aus der Katalysatorschüttung befindet. Derartige Aktivitätsstrukturierungen des Katalysators beschreiben beispielsweise die DE 198 23 262, DE 198 23 275, DE 100 40 827 und DE 102 06 989.

Insbesondere bei höheren Kohlenwasserstoffbeladungen wird es allerdings zunehmend schwieriger, eine ausreichend gute Produktqualität ohne Ausbeuteverluste im Reaktor durch Totaloxidation zum CO und CO₂ zu erhalten, da hier die aktivste, aber unselektivste Katalysatorschicht am Gasaustritt der Katalysatorschüttung einen höheren Beitrag zum Gesamtumsatz leisten muss. Dieser Effekt tritt über die Zeit auch durch die langsame Desaktivierung der im Allgemeinen stark belasteten Selektivschicht oder Selektivschichten am Gaseintritt der Katalysatorschüttung auf.

Eine Möglichkeit zur Überwindung der zuvor beschriebenen Schwierigkeiten besteht in der Verwendung von zwei getrennten Reaktoren anstelle eines einzigen Reaktors. So beschreibt beispielsweise die DE 20 05 969 ein Verfahren zur Herstellung von Phthalsäureanhydrid in zwei voneinander getrennten Reaktoren. Beim ersten Reaktor handelt es sich um einen salzbadgekühlten und weitgehend isotherm betriebenen Rohrbündelreaktor, während es sich bei dem zweiten Reaktor um einen adiabat betriebenen Schachtofen handelt. Die Reaktion im ersten Reaktor wird dabei so geführt, dass 1 bis 20 Gew.-% des eingesetzten Kohlenwasserstoffs unverändert bleiben. Des Weiteren ist das Verfahren dadurch gekennzeichnet, dass die Temperatur T₁ des Wärmetauschermittels im ersten Reaktor zwischen 380 und 430 °C liegt und die Eingangstemperatur T₂ der adiabat betriebenen zweiten Reaktionsstufe der Beziehung T₂ = T₁ -5 bis 150 °C gehorcht.

Das in der DE 198 07 018 und der US 5,969,160 beschriebene Verfahren umfasst mindestens zwei getrennte Reaktoren, wobei es sich bei dem ersten Reaktor um einen salzbadgekühlten Hauptreaktor handelt und bei dem zweiten Reaktor um einen Nachreaktor ohne Kühleinrichtung mit dem gleichen oder einem unterschiedlichen Katalysator. Dabei erfolgt der Produktgasstrom im Nachreaktor von oben nach unten. Das Verfahren ermöglicht ein Absenken der Temperatur im Hauptreaktor in Richtung zu Unteroxidationsbedingungen, weshalb die Phthalid-Werte im Hauptreaktorausgang bzw. Nachreaktoreingang im Bereich von 0,5 bis 0,9 Gew.-% liegen. Die Steuerung dieser Bedingungen erfolgt durch die Beobachtung des o-Xylol-Gehaltes am Hauptreaktorausgang bzw. Nachreaktoreingang, welcher ausdrücklich weniger als 100 ppm (0,01 Gew.-%) betragen soll.

Ein weiteres Nachreaktorkonzept wird in H.-J. Eberle, J. Breimair, H. Domes, T. Gutermuth, PTQ Summer 2000, 129-133 beschrieben. Hier wird dem salzbadgekühlten Rohrbündelreaktor, welcher einen mehrlagigen Katalysator enthält, ein Nachreaktor mit einem Wabenkatalysator nachgeschaltet, wobei der Produktgasstrom vor Eintritt in die Nachreaktorzone über einen Produktgaskühler auf die gewünschte Eintrittstemperatur abgekühlt wird. Der Nachreaktor dient in erster Linie zur Einhaltung einer vorgegebenen Qualität des erzeugten Phthalsäureanhydrids, insbesondere bei hohen o-Xylol-Beladungen und bei gealterten Hauptreaktorkatalysatoren. Die o-Xylol-Konzentration am Nachreaktoreingang liegt mit einem Hauptreaktorkatalysator, welcher das Ende der Lebenszeit erreicht hat, bei etwa 0,65- 0,70 Gew.-% der Summe der organischen Komponenten im Produktgasstrom und die Konzentration an unteroxidierten Nebenkomponenten wie Phthalid oder o-Tolylaldehyd bei 0,20- 0,50 Gew.-% der Summe der organischen Komponenten im Produktgasstrom.

Der Erfindung liegt die Aufgabe zugrunde, die Gesamtausbeute an Phthalsäureanhydrid ohne oder ohne wesentliche Verschlechterung der Produktqualität zu erhöhen.

Die Aufgabe wird gelöst durch ein Verfahren zur Herstellung von Phthalsäureanhydrid durch katalytische Gasphasenoxidation von o-Xylol, bei dem man ein gasförmiges Gemisch von o-Xylol und einem sauerstoffhaltigen Gas in einem Hauptreaktor zu einem gasförmigen intermediären Reaktionsprodukt umsetzt, das nicht umgesetztes o-Xylol, Phthalsäureanhydrid-Unteroxidationsprodukte und Phthalsäureanhydrid enthält, wobei man die im Hauptreaktor entstehende Reaktionswärme zumindest teilweise durch indirekte Kühlung mit einem Wärmeträgermedium abführt, und das intermediäre Reaktionsprodukt mit einer Temperatur, die niedriger ist als die tiefste Temperatur des Wärmeträgermediums in einen Nachreaktor einführt, das dadurch gekennzeichnet ist, dass die Konzentration an nicht umgesetztem o-Xylol im intermediären Reaktionsprodukt wenigstens 1 Gew.-%, und die Summe der Konzentrationen von Phthalsäureanhydrid-Unteroxidationsprodukten im intermediären Reaktionsprodukt wenigstens 0,5 Gew.-% beträgt, jeweils bezogen auf das Gesamtgewicht der organischen Komponenten im intermediären Reaktionsprodukt.

Unter Phthalsäureanhydrid-Unteroxidationsprodukten werden C₈-Körper mit einer niedrigeren Oxidationsstufe als Phthalsäureanhydrid verstanden, die zu Phthalsäureanhydrid weiter oxidierbar sind. Hierzu zählen insbesondere o-Tolylaldehyd, o-Tolylsäure und Phthalid.

Typischerweise beträgt die Konzentration an nicht umgesetztem o-Xylol im intermediären Reaktionsprodukt 1,2 bis 5,0 Gew.-%, stärker bevorzugt 1,4 bis 2,0 Gew.-%.

Vorzugsweise beträgt die Summe der Konzentrationen von o-Tolylaldehyd und Phthalid im intermediären Reaktionsprodukt wenigstens 0,5 Gew.-%, z. B. 0,6 bis 1,5 Gew.-%, stärker bevorzugt 0,7 bis 1,3 Gew.-%.

Vorzugsweise beträgt die Konzentration an o-Tolylaldehyd im intermediären Reaktionsprodukt wenigstens 0,25 Gew.-%, insbesondere 0,35 bis 0,6 Gew.-%.

Vorzugsweise beträgt die Konzentration an Phthalid im intermediären Reaktionsprodukt wenigstens 0,25 Gew.-%, insbesondere 0,35 bis 0,6 Gew.-%.

Im Allgemeinen beträgt das Gewichtsverhältnis von nicht umgesetztem o-Xylol zu o-Tolylaldehyd im intermediären Reaktionsprodukt 1 bis 20, vorzugsweise 2 bis 10; und das Gewichtsverhältnis von nicht umgesetztem o-Xylol zu Phthalid 1 bis 20, vorzugsweise 2 bis 10.

Der Gehalt an o-Xylol im intermediären Reaktionsprodukt ist vorzugsweise kleiner oder gleich 20 g/Nm³, besonders bevorzugt kleiner oder gleich 15 g/ m³.

In bevorzugten Ausführungsformen des erfindungsgemäßen Verfahrens gewinnt man kontinuierlich oder in zeitlichen Abständen, z. B. wenigstens einmal pro Woche oder wenigstens einmal täglich, Messwerte für die Konzentration an o-Xylol im intermediären Reaktionsprodukt und bildet aus den Messwerten Regeleingriffe für die Temperatur des Wärmeträgermediums. So kann man die Temperatur des Wärmeträgermediums absenken, falls die Konzentration an o-Xylol im intermediären Reaktionsprodukt unter einen vorgegebnen Sollwert oder den erfindungsgemäßen Grenzwert fällt bzw. die Temperatur des Wärmeträgermediums erhöhen, falls die Konzentration an o-Xylol im intermediären Reaktionsprodukt zu hoch ist.

Die auf die organischen Komponenten im intermediären Reaktionsprodukt bezogene Konzentration von o-Tolylaldehyd bzw. Phthalid kann ermittelt werden, indem man bei Umgebungstemperatur (23 °C) alle bei dieser Temperatur kondensierbaren Komponenten des intermediären Reaktionsprodukts kondensiert und das Kondensat mittels Gaschromatographie in einem geeigneten Lösungsmittel, wie Aceton, analysiert. Kohlenmonoxid und/oder Kohlendioxid, die gegebenenfalls im intermediären Reaktionsprodukt enthalten sind, gelten nicht als organische Komponenten des intermediären Reaktionsprodukts.

Als Hauptreaktor findet bevorzugt ein salzbadgekühlter Röhrenreaktor Anwendung. Dieser umfasst ein von einem Wärmeträgermedium in Gestalt eines Salzbades umströmtes Rohrbündel. Die einzelnen katalysatorgefüllten Rohre enden in einem oberseitigen Rohrboden bzw. einem unterseitigen Rohrboden. Das Reaktionsgas wird im Allgemeinen von oben nach unten, d. h. in Richtung der Schwerkraft, durch die Rohre geleitet; es ist aber auch eine umgekehrte Strömungsrichtung denkbar. Beabstandet zueinander befinden sich am Mantel des Reaktors Ringkanäle, durch die das Wärmeträgermedium aus dem Reaktor abgezogen bzw. nach Passieren einer Umwälzpumpe dem Reaktor wieder zugeführt wird. Ein Teilstrom des umgewälzten Wärmeträgermediums wird durch einen Kühler geleitet, in dem z. B. Sattdampf produziert wird. Im Inneren des Reaktors können sich in üblicher Weise Leitbleche befinden, um dem Wärmeträgermedium im Bereich des Rohrbündels eine radiale Strömungskomponente zu erteilen.

Die Temperaturdifferenz des Wärmeträgermediums zwischen Reaktorein- und -austritt kann 0,5 bis 12 °C, meist 1 bis 8 °C betragen. Das Wärmeträgermedium kann in Bezug auf das Reaktionsgas sowohl im Gleichstrom als auch im Gegenstrom durch den Röhrenreaktor hindurchtreten. Ferner kann der Röhrenreaktor mehrere Reaktionszonen mit voneinander getrennten oder miteinander verbundenen Kreisläufen des Wärmeträgermediums umfassen. Als "tiefste Temperatur des Wärmeträgermediums" wird die niedrigste Temperatur bezeichnet, die das Wärmeträgermedium an einer Stelle innerhalb des Reaktormantels (typischerweise in der Nähe der Eintrittsstelle des von der Umwälzpumpe kommenden Wärmeträgermediums in den Reaktor) aufweist. Umfasst der Reaktor mehrere Reaktionszonen, ist auf die bei der niedrigsten Temperatur betriebene Zone abzustellen.

Der Hauptreaktor wird typischerweise mit einer Temperatur des Wärmeträgermediums von 320 bis 390 °C betrieben. Die Gastemperatur im Hauptreaktor liegt im Bereich von 340 bis 460 °C, besonders bevorzugt im Bereich von 360 bis 435 °C.

Das dem Katalysator zugeführte Reaktionsgas wird im allgemeinen durch Vermischen von einem molekularen Sauerstoff enthaltenden Gas, das außer Sauerstoff noch geeignete Reaktionsmoderatoren und/oder Verdünnungsmittel, wie Dampf, Kohlendioxid und/oder Stickstoff, enthalten kann, mit o-Xylol erzeugt, wobei das sauerstoffhaltige Gas im allgemeinen 1 bis 100 mol-%, vorzugsweise 2 bis 50 mol-% und besonders bevorzugt 10 bis 30 mol-% Sauerstoff, 0 bis 30 mol-%, vorzugsweise 0 bis 10 mol-% Wasserdampf sowie 0 bis 50 mol-%, vorzugsweise 0 bis 1 mol-% Kohlendioxid, Rest Stickstoff, enthalten kann. Das sauerstoffhaltige Gas ist in der Regel Luft.

Die o-Xylol-Beladung des in den Hauptreaktor eintretenden gasförmigen Gemisches beträgt in der Regel 30 g bis 150 g je Nm³ Gas, vorzugsweise wenigstens 60 g/Nm³, z. B. 75 bis 120 g/Nm³.

Der Hauptreaktor ist mit einem Katalysator oder aufeinanderfolgenden Schichten verschiedener Katalysatoren befüllt, die zur Katalyse der Gasphasenoxidation von o-Xylol zu Phthalsäureanhydrid geeignet sind. Vorzugsweise handelt es sich um Schalen- oder Kugelkatalysatoren, auf deren Kern aus einem inerten Trägermaterial eine oder mehrere Schichten aus katalytisch aktiven Metalloxiden aufgebracht sind.

Bevorzugt werden im Hauptreaktor Katalysatoren eingesetzt, deren Aktivität zu Gunsten einer höheren Selektivität reduziert ist. Unter Selektivität wird hier die Selektivität bezüglich der Summe von Phthalsäureanhydrid und aller C₈-Körper verstanden, die zu Phthalsäureanhydrid weiter oxidierbar sind. In aktivitätsstrukturierten Katalysatorschüttungen, wie den eingangs erwähnten, sind dies in der Regel die gaseintrittseitig gelegenen Katalysatoren. Sie werden häufig als "Selektivkatalysatoren" bezeichnet, im Unterschied zu den gasaustrittsseitig gelegenen "Aktivkatalysatoren". Der Hauptreaktor enthält vorzugsweise nur eine oder mehrere Selektivkatalysatorschichten und keine typische Aktivkatalysatorschicht. Hierdurch resultieren zwar im intermediären Reaktionsprodukt höhere Konzentrationen an nicht umgesetztem o-Xylol und unteroxidierten Nebenkomponenten, jedoch kann im Hauptreaktor die Oxidation von o-Xylol zu Phthalsäureanhydrid (bzw. zu C₈-Körper, die im Nachreaktor zu Phthalsäureanhydrid oxidierbar sind) selektiver betrieben werden. So kann die Temperatur des Wärmeträgermediums bei alterndem Katalysator besser angepasst werden, da sich die Katalysatorschicht oder die Katalysatorschichten im Hauptreaktor - anders als bei einer Schüttung aus Selektiv- und Aktivkatalysator - nur wenig hinsichtlich ihrer Aktivitäten unterscheiden.

Typische derartige Selektivkatalysatoren, die man im Hauptreaktor vorzugsweise ausschließlich verwendet, sind
a) ein oder mehrere Katalysatoren, deren Aktivmasse ein Silber, Vanadium und gegebenenfalls ein oder mehrere Promotormetalle enthaltendes Multimetallmischoxid, insbesondere eine Silber-Vanadiumoxid-Bronze, umfasst, und/oder
b) ein oder mehrere Katalysatoren auf der Basis von Vanadiumoxid und Titandioxid, worin der Alkalimetallgehalt der Aktivmasse größer oder gleich 0,12 Gew.-% und der Phosphorgehalt der Aktivmasse (gerechnet als P) kleiner oder gleich 0,20 Gew.-% ist, und
c) Kombinationen von einem oder mehreren Katalysatoren gemäß der vorstehenden Definition a) und einem oder mehreren Katalysatoren gemäß der vorstehenden Definition b).

Multimetallmischoxide, die Silber, Vanadium und gegebenenfalls ein oder mehrere Promotormetalle enthalten, sowie Silber-Vanadiumoxid-Bronzen und ihre Herstellung sind an sich bekannt, z. B. aus der WO 00/27753, WO 01/85337 und WO 2005/012216. Unter Silber-Vanadiumoxid-Bronzen werden Silber-Vanadiumoxid-Verbindungen mit einem atomaren Ag : V-Verhältnis von weniger als 1 verstanden. Es handelt sich im Allgemeinen um halbleitende oder metallisch leitfähige, oxidische Festkörper, die bevorzugt in Schicht- oder Tunnelstrukturen kristallisieren, wobei das Vanadium im [V₂O₅]-Wirtsgitter teilweise reduziert zu V(IV) vorliegt. Silber-Vanadiumoxid-Bronzen bilden sich oberhalb 200 °C, insbesondere bei Temperaturen von mehr als 300 °C, durch Zersetzung der Multimetallmischoxide.

Geeignet sind z. B. Multimetallmischoxide der allgemeinen Formel I

Ag_{a-c}M¹V₂O_{d} * e H₂O, I

worin
- a: einen Wert von 0,3 bis 1,9 hat,
- M¹: für wenigstens ein unter Alkali- und Erdalkalimetallen, Bi, TI, Cu, Zn, Cd, Pb, Cr, Au, Al, Fe, Co, Ni, Mo, Nb, Ce, W, Mn, Ta, Pd, Pt, Ru und/oder Rh ist ausgewähltes Metall steht,
- c: einen Wert von 0 bis 0,5 hat, mit der Maßgabe, dass (a-c) ≥ 0,1 ist,
- d: eine Zahl, die sich durch die Wertigkeit und Häufigkeit der von Sauerstoff verschiedenen Elemente in der Formel I bestimmt, bedeutet und
- e: einen Wert von 0 bis 20, vorzugsweise 0 bis 5, hat.

Im Multimetalloxid der Formel I hat die Variable a vorzugsweise einen Wert von 0,5 bis 1,0 und besonders bevorzugt 0,6 bis 0,9, der Wert der Variablen b beträgt vorzugsweise 0 bis 0,1, und der Wert der Variablen c beträgt vorzugsweise 0,005 bis 0,2, insbesondere 0,01 bis 0,1.

Die Zahl d bestimmt sich aus der Wertigkeit und Häufigkeit der von Sauerstoff verschiedenen Elemente im Multimetalloxid der Formel I. Die Zahl e, die ein Maß für den Wassergehalt ist, beträgt vorzugsweise 0 bis 5.

M¹ steht vorzugsweise für Na, K, Rb, TI, Ni, W, Co, Fe, Mo, Nb, Zn, Ce und Mn.

Besonders bevorzugt sind Multimetalloxide der allgemeinen Formel la

AgₐV₂O_{d} * e H₂O, Ia

worin
- a: einen Wert von 0,6 bis 0,9 hat,
- d: die oben angegebene Bedeutung hat, und
- e: einen Wert von 0 bis 5 hat.

Zur Herstellung der Multimetalloxide wird im Allgemeinen eine Suspension von Vanadiumpentoxid (V₂O₅) mit der Lösung einer Silberverbindung sowie gegebenenfalls einer Lösung einer Verbindung der Metallkomponente M¹ und einer Verbindung von Q erhitzt. Als Lösungsmittel für diese Umsetzung wird bevorzugt Wasser verwendet. Als Silbersalz wird bevorzugt Silbernitrat verwendet, die Verwendung anderer löslicher Silbersalze, z. B. Silberacetat, Silberperchlorat oder Silberfluorid ist ebenfalls möglich.

Als Salze der Metallkomponente M¹ werden in der Regel solche gewählt, die im verwendeten Lösungsmittel löslich sind. Wird Wasser als Lösungsmittel bei der Herstellung der erfindungsgemäßen Multimetalloxide verwendet, können beispielsweise die Perchlorate oder Carboxylate, insbesondere die Acetate, der Metallkomponente M¹ eingesetzt werden. Bevorzugt werden die Nitrate der betreffenden Metallkomponente M¹ verwendet.

Je nach der gewünschten chemischen Zusammensetzung des Multimetalloxids der Formel I werden zu dessen Herstellung die sich aus a und c von Formel I ergebenden Mengen von V₂O₅, Silberverbindung sowie der Verbindung der Metallkomponente M¹ miteinander umgesetzt. Das so gebildete Multimetalloxid kann aus der Reaktionsmischung isoliert und bis zur weiteren Verwendung gelagert werden. Besonders vorteilhaft wird die Isolierung der erhaltenen Multimetalloxid-Suspension mittels Sprühtrocknung durchgeführt. Das sprühgetrocknete Pulver wird dann auf einen inerten Träger aufgebracht.

Katalysatoren auf der Basis von Vanadiumoxid und Titandioxid enthalten neben Titandioxid (in Form seiner Anatasmodifikation) Vanadiumpentoxid. Typische Katalysatoren auf der Basis von Vanadiumoxid und Titandioxid und ihre Herstellung sind in der DE 198 23 262 beschrieben.

Vorzugsweise enthält der Katalysator auf der Basis von Vanadiumoxid und Titandioxid im calzinierten Zustand 1 bis 20 Gew.-% Vanadiumoxid, berechnet als V₂O₅, und 80 bis 99 Gew.-% Titandoxid, berechnet als TiO₂. Des Weiteren können in geringen Mengen eine Vielzahl anderer oxidischer Verbindungen enthalten sein, die als Promotoren die Aktivität und Selektivität des Katalysators beeinflussen. Als die Aktivität vermindernden und die Selektivität erhöhenden Promotoren werden in der Regel Alkalimetalle, wie Cäsium, Lithium, Kalium und Rubidium, und insbesondere Cäsium verwendet.

Als die Aktivität erhöhende Zusätze werden in der Regel Phosphorverbindungen verwendet. In den Selektivkatalysatoren werden keine oder allenfalls geringe Zusätze an Phosphorverbindungen verwendet.

Die Katalysatoren auf der Basis von Vanadiumoxid und Titandioxid können außerdem Antimonverbindungen enthalten.

Die Komponenten werden in Form deren Oxide oder in Form von Verbindungen eingesetzt, welche sich beim Erhitzen bzw. beim Erhitzen in Gegenwart von Sauerstoff in Oxide umwandeln. Als Vanadium-Komponente können Vanadiumoxide oder Vanadiumverbindungen, die sich beim Erhitzen in Vanadiumoxide umwandeln, einzeln oder in Form deren Gemische eingesetzt werden. Vorzugsweise werden V₂O₅ oder NH₄VO₃ eingesetzt. Man kann auch ein Reduktionsmittel, wie Ameisensäure oder Oxalsäure, mitverwenden, um die Vanadium(V)-Verbindung zumindest teilweise zu Vanadium(IV) zu reduzieren. Geeignete Promotor(vorläufer)verbindungen sind die entsprechenden Oxide oder Verbindungen, die sich nach dem Erhitzen in Oxide umwandeln, wie Sulfate, Nitrate, Carbonate. Geeignet sind beispielsweise Na₂CO₃, K₂O, Cs₂O, Cs₂CO₃, Cs₂SO₄, P₂O₅, (NH₄)₂HPO₄, Sb₂O₃.

Zur Bildung der Aktivmasse bereitet man im Allgemeinen eine wässrige Aufschlämmung der Verbindung der Vanadium-Komponente, des Titandioxids sowie von Promotor(vorläufer)verbindungen in geeigneten Mengen und rührt die Aufschlämmung, bis eine ausreichende Homogenisierung erreicht ist. Die Aufschlämmung kann dann sprühgetrocknet werden oder als solche zur Beschichtung verwendet werden.

Bei den im erfindungsgemäßen Verfahren verwendeten Katalysatoren handelt es sich im Allgemeinen um Schalenkatalysatoren, bei denen die katalytisch aktive Masse schalenförmig auf einem inerten Träger aufgebracht ist. Die Schichtdicke der katalytisch aktiven Masse beträgt in der Regel 0,02 bis 0,2 mm, vorzugsweise 0,05 bis 0,1 mm. Im Allgemeinen weisen die Katalysatoren eine schalenförmig aufgebrachte Aktivmasseschicht mit im Wesentlichen homogener chemischer Zusammensetzung auf.

Als inertes Trägermaterial können praktisch alle bekannten Trägermaterialien eingesetzt werden, Verwendung finden, beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Das Trägermaterial ist in der Regel nicht-porös. Als vorteilhafte Trägermaterialien sind insbesondere Steatit und Siliciumcarbid hervorzuheben. Die Form des Trägermaterials ist im Allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern. Bevorzugt wird Steatit in Form von Kugeln mit einem Durchmesser von 3 bis 6 mm oder von Ringen mit einem äußeren Durchmesser von 5 bis 9 mm und einer Länge von 4 bis 7 mm verwendet.

Die Aufbringung der Aktivmasseschicht auf den Träger kann mit beliebigen an sich bekannten Methoden erfolgen, z. B. durch Aufsprühen von Lösungen oder Suspensionen in der Dragiertrommel oder Beschichtung mit einer Lösung oder Suspension in einer Wirbelschicht. Dabei können der katalytisch aktiven Masse organische Binder, bevorzugt Copolymere, vorteilhaft in Form einer wässrigen Dispersion, von Vinylacetat/Vinyllaurat, Vinylacetat/Acrylat, Styrol/Acrylat, Vinylacetat/Maleat, Vinylacetat/Ethylen sowie Hydroxyethylcellulose zugesetzt werden, wobei mit Vorteil Bindermengen von 3 bis 20 Gew.- %, bezogen auf den Feststoffgehalt der Lösung der Aktivmassenbestandteile, eingesetzt werden. Die aufgetragenen Bindemittel brennen nach dem Einfüllen des Katalysators und Inbetriebnahme des Reaktors innerhalb kurzer Zeit aus. Der Binderzusatz hat zudem den Vorteil, dass die Aktivmasse gut auf dem Träger haftet, so dass Transport und Einfüllen des Katalysators erleichtert werden.

Das den Hauptreaktor verlassende gasförmige intermediäre Reaktionsprodukt wird einem Nachreaktor zugeführt. Es bildet den ausschließlichen Zulauf zum Nachreaktor, d. h. dem intermediären Reaktionsprodukt werden keine Gase, wie Frisch- oder Abluft oder o-Xylol, beigemischt.

Als Nachreaktor kommen alle Reaktoren in Betracht, die zur Durchführung heterogen katalysierter Gasphasenreaktionen geeignet sind, insbesondere Festbettreaktoren mit fest angeordneter Katalysatorschüttung, Rohrbündelreaktoren, Reaktoren mit katalysatorbeschichteten monolithischen Wabenkörpern und dergleichen. Es kann sich um vertikale oder liegende Bauformen handeln; vertikale Reaktoren können von unten oder von oben angeströmt werden.

Das intermediäre Reaktionsprodukt tritt mit einer Temperatur in den Nachreaktor ein, die niedriger ist als die tiefste Temperatur des Wärmeträgermediums im Hauptreaktor. Meist ist es bevorzugt, dass man das aus dem Hauptreaktor austretende intermediäre Reaktionsprodukt vor dem Eintritt in den Nachreaktor mittels einer geeigneten Kühlstufe kühlt. Die Differenz zwischen der Austrittstemperatur aus dem Hauptreaktor und der Eintrittstemperatur in den Nachreaktor beträgt dabei vorzugsweise wenigstens 5 °C, insbesondere wenigstens 10 °C. Die Leistung der Kühlstufe wird zweckmäßigerweise in Abhängigkeit von der o-Xylol-Konzentration im intermediären Reaktionsprodukt und der Aktivität des Nachreaktor-Katalysators so gesteuert, dass die Phthalsäureanhydrid-Ausbeute und -Reinheit maximiert werden.

Der Hauptreaktor, die Kühlstufe und der Nachreaktor können dabei in getrennten Apparaten angeordnet sein. Als Kühlstufe eignen sich flüssigkeitsgekühlte indirekte Wärmetauscher oder Gas-Gas-Wärmetauscher, über die das dem Hauptreaktor zugeführte Reaktionsgas vorgewärmt werden kann.

Alternativ können aber auch Hauptreaktor und Kühlstufe oder Kühlstufe und Nachreaktor in einem einzigen Apparat kombiniert werden. Beispielsweise kann die Kühlstufe als unmittelbare Verlängerung der Reaktionsrohre des Hauptreaktors ausgebildet sein, die im Bereich der Kühlstufe nicht mit Katalysator gefüllt sind und mittels eines getrennten Kreislaufs eines Wärmeträgermediums oder mit einem Teilstrom des vom Kühler kommenden Wärmeträgermediums gekühlt werden.

Es können auch die Funktionen des Hauptreaktors, einer Kühlstufe und des Nachreaktors in einem einzigen Gehäuse angeordnet sein, wie in der DE 101 44 857 beschrieben.

Die Bauweise des Nachreaktors richtet sich nach dessen Betriebsweise. In einer möglichen Ausführungsform betreibt man den Nachreaktor im Wesentlichen adiabatisch.

In einer anderen möglichen Ausführungsform führt man zumindest einen Teil der im Nachreaktor entstehenden Reaktionswärme durch indirekte Kühlung mit einem Wärmeträgermedium ab. Geeignete Wärmeträgermedien für den Nachreaktor sind Wärmeträgeröle, Salzschmelzen, Luft oder Wasser. Typische Kühlverfahren sind in der prioritätsälteren Patentanmeldung DE 10 2004 061770 zu finden. Besonders bevorzugt ist eine Bauweise bei dem das Reaktionsgas zunächst eine adiabate Katalysatorschicht und anschließend eine zwischen Thermoblechplatten geschüttete Katalysatorschicht durchströmt.

Als Nachreaktorkatalysator werden bevorzugt Schalen- oder Kugelkatalysatoren verwendet, auf deren Kern aus einem inerten Trägermaterial eine oder mehrere Schichten aus katalytisch aktiven Metalloxiden aufgebracht sind. Typische Trägermaterialien und typische eingesetzte Metalloxide und Promotoren sind in der DE 198 23 262 zu finden. Es werden vorzugsweise Katalysatoren verwendet, die von den im Hauptreaktor verwendeten Katalysatoren verschieden sind. Bevorzugt werden "Aktivkatalysatoren" eingesetzt. Daher verwendet man im Nachreaktor vorzugsweise wenigstens einen Katalysator auf der Basis von Vanadiumoxid und Titandioxid, worin der Alkalimetallgehalt der Aktivmasse kleiner oder gleich 0,20 Gew.-%. Optional enthält die Aktivmasse Phosphor; der Phosphorgehalt der Aktivmasse ist beispielsweise größer oder gleich 0,12 Gew.-%.

Der Nachreaktor wird typischerweise bei Gastemperaturen von 240 bis 360 °C, besonders bevorzugt bei 270 bis 350 °C betrieben. Die höchste im Nachreaktor auftretende Gastemperatur ist vorzugsweise um wenigstens 30 °C, insbesondere wenigstens 40 °C niedriger als die höchste im Hauptreaktor auftretende Gastemperatur.

Im Allgemeinen wird das den Nachreaktor verlassende Reaktionsgas in einem Produktgaskühler abgekühlt und das Phthalsäureanhydrid aus den heißen Reaktionsgasen in üblicher Weise mittels alternierend betriebener Phthalsäureanhydrid-Abscheider abgeschieden werden. Optional kann den Abscheidern ein so genannter Flüssigabscheider vorgeschaltet sein, was besonders bei hohen Beladungen vorteilhaft ist.

Das erfindungsgemäße Verfahren ist auch übertragbar auf die Herstellung anderer Produkte durch katalytische Gasphasenoxidation, wie beispielsweise Phthalsäureanhydrid aus Naphthalin oder o-Xylol/Naphthalin-Gemischen, Acrylsäure aus Propen, Maleinsäureanhydrid aus Benzol, Butan, Buten oder Butadien, Benzoesäure aus Toluol, usw.

Die Erfindung wird durch die folgenden Beispiele und Vergleichsbeispiele näher veranschaulicht.

### Beispiele

### Herstellung der Katalysatoren

### Herstellung des Hauptreaktorkatalysators I (Selektivkatalysator)

Nach 18-stündigem Rühren werden in einer Dragiertrommel 236,6 g einer Suspension, bestehend aus 104,9 g Oxalsäure, 39,4 g Vanadiumpentoxid, 17,0 g Antimonoxid, 2,87 g Cäsiumsulfat, 3,15 g Ammoniumdihydrogenphosphat, 149,0 g Formamid, 465,9 g Titandioxid der Anatas-Modifikation mit einer BET-Oberfläche von 20 m²/g und 721,0 g Wasser bei 160 °C zusammen mit 13,0 g organischem Binder auf 1400 g Steatitringe der Dimension 8 x 6 x 5 mm (Außendurchmesser x Höhe x Innendurchmesser) aufgebracht. In einem zweiten Schritt werden die so beschichteten Ringe mit 236,2 g einer zweiten Suspension, die zuvor ebenfalls 18 h gerührt wurde, bestehend aus 56,7 g Oxalsäure, 21,0 g Vanadiumpentoxid, 2,87 g Cäsiumsulfat, 198,0 g Formamid, 501,9 g Titandioxid und 720,3 g Wasser zusammen mit 12,8 g organischem Binder beschichtest.

Nach Kalzination des Katalysators für eine Stunde bei 450 °C beträgt die auf die Steatitringe aufgebrachte Aktivmasse 9,3 %. Die Aktivmasse besitzt die Zusammensetzung 5,75 % V₂O₅,1,6 % Sb₂O₃, 0,40 % Cs, 0,08 % P, Rest TiO₂.

Herstellung des Hauptreaktorkatalysators II (Aktivkatalysator)

Nach 18-stündigem Rühren werden in einer Dragiertrommel 538,0 g einer Suspension, bestehend aus 106,4 g Oxalsäure, 39,4 g Vanadiumpentoxid, 17,0 g Antimonoxid, 0,63 g Cäsiumsulfat, 3,35 g Ammoniumdihydrogenphosphat, 149,6 g Formamid, 467,5 g Titandioxid der Anatas-Modifikation mit einer BET-Oberfläche von 20 m²/g und 719,1 g Wasser bei 160 °C auf 1400 g Steatitringe der Dimension 8 x 6 x 5 mm (Außendurchmesser x Höhe x Innendurchmesser) aufgebracht.

Nach Kalzination des Katalysators für eine Stunde bei 450 °C beträgt die auf die Steatitringe aufgebrachte Aktivmasse 10,5 %. Die Aktivmasse besitzt die Zusammensetzung 7,5 % V₂O₅, 3,2 % Sb₂O₃, 0,09 % Cs, 0,17 % P, Rest TiO₂.

### Herstellung des Hauptreaktorkatalysators III (Selektivkatalysator mit AgV-Bronze)

Der Katalysator mit einer Aktivmasse der Zusammensetzung Ce_{0,02}Ag_{0,71}V₂Oₓ wurde gemäß WO 2005/012216 (Katalysator A.1) hergestellt.

### Herstellung des Nachreaktorkatalysators

Nach 18-stündigem Rühren werden in einer Dragiertrommel 540,2 g einer Suspension, bestehend aus 105,5 g Oxalsäure, 39,4 g Vanadiumpentoxid, 17,0 g Antimonoxid, 0,29 g Cäsiumsulfat, 8,9 g Ammoniumdihydrogenphosphat, 149,0 g Formamid, 467,0 g Titandioxid der Anatas-Modifikation mit einer BET-Oberfläche von 20 m²• g⁻¹ und 720,5 g Wasser bei 160 °C auf 1400 g Steatitringe der Dimension 8 x 6 x 5 mm (Außendurchmesser x Höhe x Innendurchmesser) aufgebracht.

Nach Kalzination des Katalysators für eine Stunde bei 450 °C beträgt die auf die Steatitringe aufgebrachte Aktivmasse 10,6 %. Die Aktivmasse besitzt die Zusammensetzung 7,5 % V₂O₅ 3,2 % Sb₂O₃ 0,04 % Cs, 0,41 % P, Rest TiO₂.

### Beispiele 1 bis 15

Als Hauptreaktor verwendete man einen Rohrbündelreaktor mit 99 Normalrohren und 2 Thermorohren. Die Normalrohre hatten eine lichte Weite von 25 mm, die Thermorohre eine lichte Weite von 29 mm mit Hülsen (10 mm Durchmesser) mit eingebautem 30-fach-Multielement mit Temperaturmessstellen im Abstand von 10 cm oder mit Probenahme-Element gemäß DE 101 10 847. Von unten nach oben wurden 320 cm Hauptreaktorkatalysator **I** in jedes der 360 cm langen Eisenrohre eingefüllt. Mittels Druckabgleich wurde dafür gesorgt, dass an jedem Rohreingang der gleiche Eingangsdruck anlag. Gegebenenfalls wurde bei den 99 Normalrohren noch etwas Hauptreaktorkatalysator I hinzugefügt oder abgesaugt; bei den beiden Thermorohren wurde der Druckabgleich durch Zusatz von Inertmaterial in Form von Steatitkugeln und Quarzkies erreicht. Die Eisenrohre waren zur Temperaturregelung von einer Salzschmelze umgeben, die sich in zwei getrennten Salzbädern befand. Das untere Salzbad (Salzbad B) umgab die Rohre vom unteren Rohrboden bis zu einer Höhe von 140 cm, das obere Salzbad (Salzbad A) umgab die Rohre von der Höhe 140 cm bis zum oberen Rohrboden.

Der Nachreaktor (Innendurchmesser 45 cm, Höhe etwa 100 cm) war auf einer Höhe von etwa 90 cm mit Kühlschlangen (Durchmesser 12 mm, Kühlschlangenabstand etwa 30 mm) ausgestattet, welche im unteren Teil über eine Höhe von 47 cm mit Luft mit Umgebungstemperatur als Kühlmedium, beaufschlagt wurden. Der Nachreaktor wurde mit Nachreaktorkatalysator bis zu einer Füllhöhe von 65 cm gefüllt.

Zum Betrieb der Anlage wurde ein Luft/o-Xylol-Gemisch bei etwa 200-205 °C Hauptreaktoreingangstemperatur von oben nach unten durch den Hauptreaktor geleitet, anschließend in einem Wärmetauscher auf eine bestimmte Nachreaktoreingangstemperatur abgekühlt und anschließend über den Nachreaktor von oben nach unten geleitet. Die Luftkühlung im unteren Teil des Nachreaktors erfolgte über eine Höhe von 47 cm so, dass sich eine bestimmte Temperatur in der Katalysatorschüttung, welche 10 cm oberhalb des Nachreaktorausgangs gemessen wurde, einstellte. Das eingesetzte o-Xylol hatte eine Reinheit von 98,5-99,0 Gew.-%.

Nach einer allgemein üblichen Hochfahrzeit des Hauptreaktorkatalysators konnten die in Tabelle 1 angegebenen Ergebnisse erhalten werden.

### Beispiele 16 bis 21

Man verwendete einen Hauptreaktor wie in den vorstehenden Beispielen. Von unten nach oben wurden 240 cm Hauptreaktorkatalysator I, 84 cm Hauptreaktorkatalysator III und anschließend 12 cm Steatitringe der Dimension 8 x 6 x 5 mm (Außendurchmesser x Höhe x Innendurchmesser) als Inertmaterial in jedes der 360 cm langen Eisenrohre eingefüllt. Mittels Druckabgleich wurde dafür gesorgt, dass an jedem Rohreingang der gleiche Eingangsdruck anlag.

Der Nachreaktor (Innendurchmesser 45 cm, Höhe ca. 100 cm) war auf einer Höhe von etwa 90 cm mit Kühlschlangen (Durchmesser 12 mm, Kühlschlangenabstand ca. 30 mm) ausgestattet, welche im unteren Teil über eine Höhe von 47 cm mit Luft mit Umgebungstemperatur als Kühlmedium beaufschlagt wurden. Der Nachreaktor wurde mit Nachreaktorkatalysator bis zu einer Füllhöhe von 65 cm gefüllt.

Zum Betrieb der Anlage wurde ein Luft/o-Xylol-Gemisch bei etwa 200- 205 °C Hauptreaktoreingangstemperatur von oben nach unten durch den Hauptreaktor geleitet, anschließend in einem Wärmetauscher auf eine bestimmte Nachreaktoreingangstemperatur abgekühlt und anschließend über den Nachreaktor von oben nach unten geleitet. Die Luftkühlung im unteren Teil des Nachreaktors erfolgte über eine Höhe von 47 cm so, dass sich eine bestimmte Temperatur in der Katalysatorschüttung, welche 10 cm oberhalb des Nachreaktorausgangs gemessen wurde, einstellte.

Nach einer allgemein üblichen Hochfahrzeit des Hauptreaktorkatalysators konnten die in Tabelle 1 angegebenen Ergebnisse erhalten werden

### Vergleichsbeispiele 1 bis 9

Man verwendete einen Hauptreaktor wie in den vorstehenden Beispielen. Von unten nach oben wurden jeweils 101 cm Hauptreaktorkatalysator II und anschließend 229 cm Hauptreaktorkatalysator I in jedes der 360 cm langen Eisenrohre eingefüllt. Mittels Druckabgleich wurde dafür gesorgt, dass an jedem Rohreingang der gleiche Eingangsdruck anlag. Gegebenenfalls wurde bei den 99 Normalrohren noch etwas Hauptreaktorkatalysator I hinzugefügt oder abgesaugt; bei den beiden Thermorohren wurde der Druckabgleich durch Zusatz von Inertmaterial in Form von Steatitkugeln und Quarzkies erreicht.

Man verwendete einen Nachreaktor wie in den vorstehenden Beispielen; durch die Kühlschlangen wurde jedoch keine Luft zur Kühlung geleitet.

Zum Betrieb der Anlage wurde ein Luft/o-Xylol-Gemisch bei etwa 200-205 °C Hauptreaktoreingangstemperatur von oben nach unten durch den Hauptreaktor geleitet, anschließend in einem Wärmetauscher auf eine bestimmte Nachreaktoreingangstemperatur abgekühlt und anschließend über den adiabat betriebenen Nachreaktor von oben nach unten geleitet. Die angegebene Temperatur wurde 10 cm oberhalb des Nachreaktorausgangs gemessen. Das eingesetzte o-Xylol hatte eine Reinheit von 98,5-99,0 Gew.-%.

Nach einer allgemein üblichen Hochfahrzeit des Hauptreaktorkatalysators konnten die in Tabelle 2 angegebenen Ergebnisse erhalten werden.

### In den Tabellen bedeuten:

Lauftag = Betriebstag ab dem ersten Start-up des Hauptreaktorkatalysators;
Salzbad A = Sälzbadtemperatur* des zum Reaktoreingang hin gelegenen Salzbades;
Salzbad B = Salzbadtemperatur* des zum Reaktorausgang hin gelegenen Salzbades (* es ist jeweils die tiefste Temperatur des Salzbades innerhalb des Reaktors angegeben);
o-Xylol HR-Ausgang, o-Tolylaldehyd HR-Ausgang bzw. Phthalid HR-Ausgang = o-Xylol- o-Tolylaldehyd- bzw. Phthalid-Gehalt in Gew.-% der organischen Komponenten des Rohproduktgases am Hauptreaktorausgang;
o-Xylol NR-Ausgang, o-Tolylaldehyd NR-Ausgang bzw. Phthalid NR-Ausgang = o-Xylol-, o-Tolylaldehyd- bzw. Phthalid-Gehalt in Gew.-% der organischen Komponenten des Rohproduktgases am Nachreaktorausgang;
PSA-Ausbeute NR-Ausgang = PSA-Ausbeute in Gew.-% bezüglich 100%igem o-Xylol aus der Analyse des Rohproduktgases im Nachreaktorausgang.

**Tabelle 1: Ergebnisse der Beispiele 1 bis 15**

| Beispiel | Lauftag | o-Xylol | Luft | Temperatur Salzbad A Salzbad B | Temperatur NR-Eingang Kat-Schüttung | o-Xylol HR-Ausgang NR-Ausgang | o-Tolylaldehyd HR-Ausgang NR-Ausgang | Phthalid HR-Ausgang NR-Ausgang | PSA-Ausbeute NR-Ausgang |
|---|---|---|---|---|---|---|---|---|---|
| | | [g/Nm³] | [Nm³/h Rohr] | [°C] | [°C] | [Gew.-%] | [Gew.-%] | [Gew.-%] | [m/m-%] |
| 1 | 60 | 63,3 | 3,8 | 350,0 | 320 | 1,41 | 0,46 | 0,47 | 116,04 |
| | | | | 350,0 | 295 | 0,04 | 0,05 | 0,09 | |
| 2 | 66 | 65,6 | 3,8 | 349,5 | 324 | 1,63 | 0,50 | 0,47 | 115,67 |
| | | | | 349,5 | 295 | 0,04 | 0,04 | 0,07 | |
| 3 | 99 | 62,8 | 3,8 | 350,0 | 320 | 1,04 | 0,35 | 0,34 | 115,34 |
| | | | | 350,0 | 291 | 0,01 | 0,02 | 0,03 | |
| 4 | 101 | 65,7 | 3,8 | 349,0 | 320 | 1,50 | 0,44 | 0,41 | 115,64 |
| | | | | 349,0 | 292 | 0,04 | 0,04 | 0,06 | |
| 5 | 103 | 66,2 | 3,8 | 349,0 | 320 | 1,51 | 0,45 | 0,42 | 115,67 |
| | | | | 349,0 | 290 | 0,04 | 0,05 | 0,07 | |
| 6 | 107 | 65,4 | 3,8 | 349,0 | 320 | 1,35 | 0,42 | 0,39 | 115,82 |
| | | | | 349,0 | 291 | 0,03 | 0,04 | 0,05 | |
| 7 | 121 | 67,9 | 3,8 | 349,5 | 322 | 1,51 | 0,46 | 0,43 | 115,66 |
| | | | | 349,5 | 290 | 0,04 | 0,04 | 0,06 | |
| 8 | 179 | 72,5 | 3,8 | 349,5 | 320 | 1,39 | 0,42 | 0,37 | 115,06 |
| | | | | 349,6 | 295 | 0,03 | 0,03 | 0,04 | |
| 9 | 180 | 73,4 | 3,8 | 349,5 | 320 | 1,45 | 0,44 | 0,38 | 115,18 |
| | | | | 349,6 | 295 | 0,04 | 0,04 | 0,05 | |
| 10 | 183 | 75,5 | 3,8 | 349,0 | 320 | 1,52 | 0,44 | 0,39 | 115,27 |
| | | | | 349,0 | 296 | 0,04 | 0,04 | 0,05 | |
| 11 | 184 | 75,5 | 3,8 | 349,0 | 320 | 1,48 | 0,42 | 0,37 | 115,12 |
| | | | | 348,9 | 296 | 0,03 | 0,04 | 0,04 | |
| 12 | 198 | 76,6 | 3,8 | 349,5 | 324 | 1,66 | 0,48 | 0,42 | 115,01 |
| | | | | 349,5 | 295 | 0,04 | 0,04 | 0,04 | |
| 13 | 199 | 76,4 | 3,8 | 349,6 | 324 | 1,58 | 0,47 | 0,42 | 115,22 |
| | | | | 349,5 | 292 | 0,04 | 0,04 | 0,05 | |
| 14 | 201 | 77,2 | 3,8 | 349,5 | 326 | 1,63 | 0,48 | 0,43 | 115,42 |
| | | | | 349,5 | 292 | 0,04 | 0,04 | 0,04 | |
| 15 | 204 | 76,6 | 3,8 | 349,5 | 326 | 1,60 | 0,47 | 0,42 | 115,20 |
| | | | | 349,5 | 291 | 0,04 | 0,04 | 0,04 | |
| | | | | | | | | | |
| 16 | 41 | 80,0 | 3,8 | 357,9 | 324 | 1,48 | 0,50 | 0,51 | 116,2 |
| | | | | 358,0 | 285 | 0,03 | 0,04 | 0,04 | |
| 17 | 42 | 80,0 | 3,8 | 357,9 | 324 | 1,14 | 0,44 | 0,49 | 116,9 |
| | | | | 358,0 | 285 | 0,03 | 0,04 | 0,04 | |
| 18 | 43 | 80,0 | 3,8 | 357,9 | 324 | 1,19 | 0,46 | 0,51 | 116,6 |
| | | | | 358,0 | 284 | 0,04 | 0,04 | 0,04 | |
| 19 | 44 | 80,0 | 3,8 | 357,9 | 320 | 1,31 | 0,48 | 0,53 | 117,0 |
| | | | | 358,0 | 290 | 0,04 | 0,04 | 0,05 | |
| 20 | 51 | 80,0 | 3,8 | 358,0 | 324 | 1,54 | 0,54 | 0,55 | 116,8 |
| | | | | 358,0 | 289 | 0,04 | 0,04 | 0,04 | |
| 21 | 52 | 80,0 | 3,8 | 357,9 | 324 | 1,56 | 0,55 | 0,56 | 116,7 |
| | | | | 358,0 | 290 | 0,04 | 0,04 | 0,04 | |

**Tabelle 2: Ergebnisse der Vergleichsbeispiele 1 bis 9**

| Vergleichs-beispiel | Lauftag | o-Xylol [g/Nm³] | Luft [Nm³/h Rohr] | Temperatur Salzbad A Salzbad B | Temperatur NR-Eingang Kat-Schüttung | o-Xylol HR-Ausgang NR-Ausgang | o-Tolylaldehyd HR-Ausgang NR-Ausgang | Phthalid HR-Ausgang NR-Ausgang | PSA-Ausbeute NR-Ausgang [m/m-%] |
|---|---|---|---|---|---|---|---|---|---|
| | | | | [°C] | [°C] | [Gew.-%] | [Gew.-%] | [Gew.-%] | |
| 1 | 148 | 60,5 | 3,8 | 354,0 | 284 | 0,02 | 0,02 | 0,01 | 113,4 |
| | | | | 353,9 | 287 | 0,01 | 0,01 | 0,00 | |
| 2 | 151 | 63,5 | 3,8 | 351,0 | 284 | 0,00 | 0,03 | 0,03 | 114,2 |
| | | | | 351,0 | 287 | 0,01 | 0,01 | 0,00 | |
| 3 | 154 | 71,0 | 3,8 | 348,0 | 284 | 0,02 | 0,05 | 0,06 | 114,5 |
| | | | | 348,0 | 287 | 0,01 | 0,02 | 0,01 | |
| 4 | 156 | 76,8 | 3,8 | 346,0 | 284 | 0,05 | 0,09 | 0,11 | 114,3 |
| | | | | 346,0 | 287 | 0,00 | 0,03 | 0,02 | |
| 5 | 157 | 80, | 3,8 | 345,0 | 284 | 0,10 | 0,14 | 0,17 | 114,1 |
| | | | | 345,1 | 288 | 0,00 | 0,04 | 0,06 | |
| 6 | 226 | 80,0 | 3,8 | 345,7 | 293 | 0,01 | 0,02 | 0,02 | 114,2 |
| | | | | 345,8 | 291 | 0,00 | 0,02 | 0,01 | |
| 7 | 247 | 80,0 | 3,8 | 346,5 | 274 | 0,10 | 0,11 | 0,14 | 114,9 |
| | | | | 346,5 | 280 | 0,00 | 0,02 | 0,03 | |
| 8 | 248 | 80,0 | 3,8 | 346,5 | 274 | 0,10 | 0,11 | 0,14 | 114,6 |
| | | | | 346,5 | 280 | 0,00 | 0,02 | 0,02 | |
| 9 | 270 | 79,9 | 3,8 | 346,7 | 274 | 0,07 | 0,09 | 0,13 | 114,5 |
| | | | | 346, | 280 | 0,00 | 0,02 | 0,02 | |

## Patentansprüche

1. Verfahren zur Herstellung von Phthalsäureanhydrid durch katalytische Gasphasenoxidation von o-Xylol, bei dem man ein gasförmiges Gemisch von o-Xylol und einem sauerstoffhaltigen Gas in einem Hauptreaktor zu einem gasförmigen intermediären Reaktionsprodukt umsetzt, das nicht umgesetztes o-Xylol, Phthalsäureanhydrid-Unteroxidationsprodukte und Phthalsäureanhydrid enthält, wobei man die im Hauptreaktor entstehende Reaktionswärme zumindest teilweise durch indirekte Kühlung mit einem Wärmeträgermedium abführt, und das intermediäre Reaktionsprodukt mit einer Temperatur, die niedriger ist als die tiefste Temperatur des Wärmeträgermediums, in einen Nachreaktor einführt, **dadurch gekennzeichnet, dass** die Konzentration an nicht umgesetztem o-Xylol im intermediären Reaktionsprodukt wenigstens 1 Gew.-%, und die Summe der Konzentrationen von Phthalsäureanhydrid-Unteroxidationsprodukten im intermediären Reaktionsprodukt wenigstens 0,5 Gew.-% beträgt, jeweils bezogen auf das Gesamtgewicht der organischen Komponenten im intermediären Reaktionsprodukt.

2. Verfahren nach Anspruch 1, wobei die Summe der Konzentrationen an o-Tolylaldehyd und Phthalid im intermediären Reaktionsprodukt wenigstens 0,5 Gew.-% beträgt.

3. Verfahren nach Anspruch 1 oder 2, wobei die Konzentration an o-Tolylaldehyd im intermediären Reaktionsprodukt wenigstens 0,25 Gew.-% beträgt.

4. Verfahren nach einem der vorhergehenden Ansprüche, wobei die Konzentration an Phthalid im intermediären Reaktionsprodukt wenigstens 0,25 Gew.-% beträgt.

5. Verfahren nach einem der vorhergehenden Ansprüche, wobei man Messwerte für die Konzentration an o-Xylol im intermediären Reaktionsprodukt gewinnt und aus den Messwerten Regeleingriffe für die Temperatur des Wärmeträgermediums im Hauptreaktor bildet.

6. Verfahren nach einem der vorhergehenden Ansprüche, wobei die o-Xylol-Beladung des in den Hauptreaktor eintretenden gasförmigen Gemisches wenigstens 60 g/Nm³ beträgt.

7. Verfahren nach einem der vorhergehenden Ansprüche, wobei der Gehalt an o-Xylol im intermediären Reaktionsprodukt kleiner oder gleich 20 g/Nm³ ist.

8. Verfahren nach einem der vorhergehenden Ansprüche, wobei die höchste im Nachreaktor auftretende Gastemperatur um wenigstens 30 °C niedriger ist als die höchste im Hauptreaktor auftretende Gastemperatur.

9. Verfahren nach einem der vorhergehenden Ansprüche, wobei man im Hauptreaktor ausschließlich Katalysatoren verwendet, die ausgewählt sind unter
a) einem oder mehreren Katalysatoren, deren Aktivmasse ein Silber, Vanadium und gegebenenfalls ein oder mehrere Promotormetalle enthaltendes Multimetallmischoxid, umfasst,
b) einem oder mehreren Katalysatoren auf der Basis von Vanadiumoxid und Titandioxid, worin der Alkalimetallgehalt der Aktivmasse größer oder gleich 0,12 Gew.-% und der Phosphorgehalt der Aktivmasse kleiner oder gleich 0,20 Gew.-% ist,
c) Kombinationen von einem oder mehreren Katalysatoren gemäß der vorstehenden Definition a) und einem oder mehreren Katalysatoren gemäß der vorstehenden Definition b).

10. Verfahren nach einem der vorhergehenden Ansprüche, wobei man im Nachreaktor wenigstens einen Katalysator auf der Basis von Vanadiumoxid und Titandioxid verwendet, worin der Alkalimetallgehalt der Aktivmasse kleiner oder gleich 0,20 Gew.-% ist.

11. Verfahren nach einem der vorhergehenden Ansprüche, wobei man zumindest einen Teil der im Nachreaktor entstehenden Reaktionswärme durch indirekte Kühlung mit einem Wärmeträgermedium abführt.

12. Verfahren nach einem der vorhergehenden Ansprüche, wobei man den Nachreaktor im Wesentlichen adiabatisch betreibt.

13. Verfahren nach einem der vorhergehenden Ansprüche, wobei man das aus dem Hauptreaktor austretende intermediäre Reaktionsprodukt vor dem Eintritt in den Nachreaktor kühlt.

## Claims

1. A process for preparing phthalic anhydride by catalytic gas phase oxidation of o-xylene, in which a gaseous mixture of o-xylene and an oxygenous gas is converted in a main reactor to a gaseous intermediate reaction product which comprises unconverted o-xylene, phthalic anhydride underoxidation products and phthalic anhydride, the heat of reaction which arises in the main reactor being removed at least partly by indirect cooling with a heat carrier medium, and the intermediate reaction product being introduced into a postreactor with a temperature which is lower than the lowest temperature of the heat carrier medium, wherein the concentration of unconverted o-xylene in the intermediate reaction product is at least 1% by weight, and the sum of the concentrations of phthalic anhydride underoxidation products in the intermediate reaction product is at least 0.5% by weight, based in each case on the total weight of the organic components in the intermediate reaction product.

2. The process according to claim 1, wherein the sum of the concentrations of o-tolylaldehyde and phthalide in the intermediate reaction product is at least 0.5% by weight.

3. The process according to claim 1 or 2, wherein the concentration of o-tolylaldehyde in the intermediate reaction product is at least 0.25% by weight.

4. The process according to any of the preceding claims, wherein the concentration of phthalide in the intermediate reaction product is at least 0.25% by weight.

5. The process according to any of the preceding claims, wherein measurements for the concentration of o-xylene are obtained in the intermediate reaction product and control interventions for the temperature of the heat carrier medium in the main reactor are formed from the measurements.

6. The process according to any of the preceding claims, wherein the o-xylene loading of the gaseous mixture which enters the main reactor is at least 60 g/m³ (STP).

7. The process according to any of the preceding claims, wherein the content of o-xylene in the intermediate reaction product is less than or equal to 20 g/m³ (STP).

8. The process according to any of the preceding claims, wherein the highest gas temperature which occurs in the postreactor is at least 30°C lower than the highest gas temperature which occurs in the main reactor.

9. The process according to any of the preceding claims, wherein the catalysts used in the main reactor are exclusively those selected from
a) one or more catalysts whose active composition is a mixed multimetal oxide comprising silver, vanadium and if appropriate one or more promoter metals,
b) one or more catalysts based on vanadium oxide and titanium dioxide, in which the alkali metal content of the active composition is greater than or equal to 0.12% by weight and the phosphorus content of the active composition is less than or equal to 0.20% by weight,
c) combinations of one or more catalysts according to the above definition a) and one or more catalysts according to the above definition b).

10. The process according to any of the preceding claims, wherein at least one catalyst based on vanadium oxide and titanium dioxide in which the alkali metal content of the active composition is less than or equal to 0.20% by weight is used in the postreactor.

11. The process according to any of the preceding claims, wherein at least some of the heat of reaction which arises in the postreactor is removed by indirect cooling with a heat carrier medium.

12. The process according to any of the preceding claims, wherein the postreactor is operated essentially adiabatically.

13. The process according to any of the preceding claims, wherein the intermediate reaction product which leaves the main reactor is cooled before it enters the postreactor.

## Revendications

1. Procédé pour la production d'anhydride phtalique par oxydation catalytique en phase gazeuse d'o-xylène, dans lequel on convertit dans un réacteur principal un mélange gazeux d'o-xylène et d'un gaz contenant de l'oxygène en un produit de réaction intermédiaire gazeux qui contient de l'o-xylène n'ayant pas réagi, des produits de sous-oxydation d'anhydride phtalique et de l'anhydride phtalique, la chaleur de réaction dégagée dans le réacteur principal étant au moins en partie dissipée par refroidissement indirect à l'aide d'un fluide caloporteur, et on introduit dans un post-réacteur le produit de réaction intermédiaire à une température qui est inférieure à la plus basse température du fluide caloporteur, **caractérisé en ce que** la concentration d'o-xylène n'ayant pas réagi dans le produit de réaction intermédiaire est d'au moins 1 % en poids, et la somme des concentrations des produits de sous-oxydation d'anhydride phtalique dans le produit de réaction intermédiaire est d'au moins 0,5 % en poids, chaque fois par rapport au poids total des composants organiques dans le produit de réaction intermédiaire.

2. Procédé selon la revendication 1, dans lequel la somme des concentrations d'o-tolylaldéhyde et de phtalide dans le produit de réaction intermédiaire est d'au moins 0,5 % en poids.

3. Procédé selon la revendication 1 ou 2, dans lequel la concentration d'o-tolylaldéhyde dans le produit de réaction intermédiaire est d'au moins 0,25 % en poids.

4. Procédé selon l'une quelconque des revendications précédentes, dans lequel la concentration de phtalide dans le produit de réaction intermédiaire est d'au moins 0,25 % en poids.

5. Procédé selon l'une quelconque des revendications précédentes, dans lequel on obtient des valeurs mesurées de la concentration d'o-xylène dans le produit de réaction intermédiaire et à partir des valeurs mesurées on établit des interventions de régulation de la température du fluide caloporteur dans le réacteur principal.

6. Procédé selon l'une quelconque des revendications précédentes, dans lequel la charge d'o-xylène du mélange gazeux entrant dans le réacteur principal est d'au moins 60 g/Nm³ normal.

7. Procédé selon l'une quelconque des revendications précédentes, dans lequel la teneur en o-xylène du produit de réaction intermédiaire est inférieure ou égale à 20 g/Nm³ normal.

8. Procédé selon l'une quelconque des revendications précédentes, dans lequel la température maximale du gaz, apparaissant dans le post-réacteur, est inférieure d'au moins 30 °C à la température maximale du gaz, apparaissant dans le réacteur principal.

9. Procédé selon l'une quelconque des revendications précédentes, dans lequel on utilise dans le réacteur principal exclusivement des catalyseurs qui sont choisis parmi
a) un ou plusieurs catalyseurs dont la matière active comprend un oxyde mixte multimétallique contenant de l'argent, du vanadium et éventuellement un ou plusieurs métaux activateurs,
b) un ou plusieurs catalyseurs à base d'oxyde de vanadium et de dioxyde de titane, dans le(s)quel(s) la teneur en métal alcalin de la matière active est supérieure ou égale à 0,12 % en poids et la teneur en phosphore de la matière active est inférieure ou égale à 0,20 % en poids,
c) des associations d'un ou plusieurs catalyseurs selon la définition a) précédente et d'un ou plusieurs catalyseurs selon la définition b) précédente.

10. Procédé selon l'une quelconque des revendications précédentes, dans lequel on utilise dans le post-réacteur au moins un catalyseur à base d'oxyde de vanadium et de dioxyde de titane, dans lequel la teneur en métal alcalin de la matière active est inférieure ou égale à 0,20 % en poids.

11. Procédé selon l'une quelconque des revendications précédentes, dans lequel on dissipe par refroidissement indirect à l'aide d'un fluide caloporteur au moins une partie de la chaleur de réaction dégagée dans le post-réacteur.

12. Procédé selon l'une quelconque des revendications précédentes, dans lequel on fait fonctionner le post-réacteur essentiellement en mode adiabatique.

13. Procédé selon l'une quelconque des revendications précédentes, dans lequel avant l'entrée dans le post-réacteur on refroidit le produit de réaction intermédiaire sortant du réacteur principal.
